# EUROPEAN PATENT APPLICATION

(11) **EP 3 922 610 A1**
(43) Date of publication of application: **15.12.2021**
(21) Application number: 20765768.5
(22) Date of filing: 04.03.2020
(51) Int. Cl.: C02F 3/10, B29C 48/00, B29C 48/91, B29C 48/92, B29K 105/00, B29K 101/12, B29K 105/04

(54) **ENVIRONMENTALLY FRIENDLY FOAMING BODY AND MANUFACTURING METHOD THEREFOR**

(30) Priority: 04.03.2019 KR 20190024506; 03.03.2020 KR 20200026559
(71) Applicant: BKT Co., Ltd., Daejeon 34109 (KR)
(72) Inventor: KIM, Seong-Ho, Gimcheon-si Gyeongsangbuk-do 39541 (KR); HYUN, Joung-Nung, Gimcheon-si Gyeongsangbuk-do 39541 (KR); KIM, Ki-Hyun, Gimcheon-si Gyeongsangbuk-do 39541 (KR); KIM, Byoung-Kyu, Gimcheon-si Gyeongsangbuk-do 39541 (KR); CHOI, In-Seok, Gimcheon-si Gyeongsangbuk-do 39541 (KR)
(74) Representative: Sander, Rolf
(86) International application number: PCT/KR2020/003029
(87) International publication number: WO 2020/180092

(57) **Abstract**

A foaming body for supporting microbes according to the present invention is superb in terms of microbial adherence to and affinity for pore areas therein, exhibits greatly improved supporting environments for microbes, and has a large surface area, thereby securing sufficient water treatment efficiency after loading microbes thereto.

## Description

### Technical Field

The present invention relates to an eco-friendly foam and a method for manufacturing the same.

### Background Art

Household water used and thrown away or industrial wastewater generated at industrial sites goes through various treatment processes. Specifically, a physical treatment through filtration, a chemical treatment using chemicals, and a biological decomposition process using microorganisms may be performed, and generally, all of these chemical treatment, physical treatment and biological treatment are used to treat wastewater. Specifically, biological decomposition using microorganisms refers to the step of decomposing organic matter mainly contained in domestic wastewater through catabolism.

Various methods may be used for this biological treatment but, to continuously treat a large amount of wastewater, microorganisms may be contained a carrier to allow the fluidized wastewater to flow and contact the microorganisms. There are various types of such carriers for containing microorganisms and specifically, ceramics, soil, or polymer resins may be used. In the case of using a polymer resin, a method for foaming the polymer may be used to expand the contact area with microorganisms.

On the other hand, in the case of a carrier in which microorganisms have been filtered, when applied to an actual water treatment facility, it should be designed to maximize the area in contact with wastewater and secure a sufficient flow rate to obtain a treatment flow rate per unit time, which has been extensively researched. For example, JP3436947B2 discloses a filtering device including a filter medium having a particle diameter of 0.5 mm to 1 mm, obtained by pulverizing porous plastic.

However, as methods of changing the structure or shape of the foam have reached their limit in effect due to many developments, it is necessary to seek other technically enhanced methods.

### (PRIOR ART DOCUMENTS)

JP3436947B2 (1994-10-04)

### Detailed Description of the Invention

### Technical Problems

An object of the present invention is to provide a foam, which is environmentally friendly and has excellent mechanical properties, such as pore properties and structural stability, as compared to conventional foamed plastics, and a method for manufacturing the same.

Another object of the present invention is to provide a foam, which has excellent adhesion and affinity for microorganisms to the pore area in the foam and has a greatly enhanced environment for containing microorganisms, and a method for manufacturing the same.

Another object of the present invention is to provide a foam capable of securing sufficient water treatment efficiency after containing microorganisms due to a large surface area and a method for manufacturing the same.

### Means to Address the Problems

According to the present invention, a method for manufacturing a foam comprises the steps of s1) preparing an extrudate by kneading and extruding a resin composition including a base resin and a natural material and s2) preparing the foam by pressurized foaming the extrudate.

In the manufacturing method according to an embodiment of the present invention, in step s1), the natural material may be a plant stem, a plant root, a plant leave, a fruit, and a fruit skin.

In the manufacturing method according to an embodiment of the present invention, in step s1), the natural material may be wheat bran, corn starch, or a mixture thereof.

In the manufacturing method according to an embodiment of the present invention, in step s1), the natural material may have a moisture content of 10% by weight or less.

In the manufacturing method according to an embodiment of the present invention, the method may further comprise the step of drying the natural material before step s1).

In the manufacturing method according to an embodiment of the present invention, in step s1), the resin composition may include 0.1 parts by weight to 10 parts by weight of the natural material with respect to 100 parts by weight of the base resin.

In the manufacturing method according to an embodiment of the present invention, in step s1), the base resin may include any one or two or more selected from the group consisting of a polyolefin-based resin, a polyvinyl chloride-based resin, a polystyrene-based resin, a polyester resin, and a copolymer thereof.

In the manufacturing method according to an embodiment of the present invention, the method may further comprise the step of performing fiber surface treatment on the natural material before step s1).

In the manufacturing method according to an embodiment of the present invention, in step s2), the pressurized foaming may be performed by introducing the extrudate, a foaming agent, and a dispersion medium into a reactor and controlling a pressure in the reactor.

In the manufacturing method according to an embodiment of the present invention, in step s2), during the pressurized foaming, a foaming temperature may be 100°C to 250°C, and a foaming pressure may be 0.5 Mpa to 10 Mpa.

In the manufacturing method according to an embodiment of the present invention, in step s2), the foaming agent may be any one or two or more selected from the group consisting of carbon dioxide, carbon monoxide, nitrogen, argon, helium, butane, and pentane.

In the manufacturing method according to an embodiment of the present invention, the foam may be a foam for containing microorganisms.

The present invention includes a foam produced by the above-described manufacturing method.

According to an embodiment of the present invention, the foam may include pores, and microorganisms may be contained in the pores.

The present invention includes a foam produced by the above-described manufacturing method, or a method for purifying wastewater using the above-described foam.

The method for purifying wastewater according to the present invention comprises the step of purifying the wastewater by bringing the wastewater in contact with the foam.

A wastewater treatment method using a foam according to an embodiment of the present invention may include a pretreatment step of precipitating and removing contaminants contained in wastewater using natural sedimentation and a purification step in which the treated water that has undergone the pretreatment step passes through a tube filled with the foam containing microorganisms.

The wastewater treatment method using the foam according to an embodiment of the present invention may include a purification step in which the wastewater containing microorganisms passes through the foam-filled tube.

### Effects of the Invention

The foam according to the present invention is environmentally friendly and has excellent mechanical properties, such as pore properties and structural stability, as compared to conventional foamed plastics.

Further, the foam according to the present invention has excellent adhesion and affinity for microorganisms to the pore area in the foam and has the effect of greatly enhancing the environment for containing microorganisms.

Further, the foam according to the present invention has a large surface area and may thus secure sufficient water treatment efficiency after containing microorganisms.

Effects which are not explicitly mentioned herein but may be expected by the technical features of the present invention, and other implicit effects are treated as described herein.

### Brief Description of the Drawings

FIG. 1 is a photograph of a foam prepared according to embodiment 1, taken on a scanning electron microscope;
FIG. 2 is an optical photograph of a foam prepared according to embodiment 1; and
FIG. 3 is a photograph of a foam prepared according to embodiment 6, taken on a scanning electron microscope.

### Best Mode to Practice the Invention

Hereinafter, an eco-friendly foam and a manufacturing method thereof according to the present invention are described in detail.

Unless otherwise defined, technical and scientific terms used herein should be interpreted as understood by one of ordinary skill in the art to which the present invention pertains. Known functions and configurations which may make the subject matter of the present invention unnecessarily unclear are skipped from the following description.

As used herein, the singular forms "a," "an," and "the" are intended to include the plural forms as well, unless the context clearly indicates otherwise.

As used herein, the terms "s1, s2, s3,..., a1, a2, a3,..., b1, b2, b3,..., a, b, c,..." are used merely to denote certain steps or means and should not be interpreted as limiting the steps or means to such order.

Unless mentioned otherwise, the symbol "%" as used herein means "% by weight."

Conventional foams typically adopt a hydrophobic polymer as a base resin and thus suffer from a significant reduction in the adhesion of microorganisms and are not suitable for the culturing environment of microorganisms. Thus, the inventors provide a foam, which has significantly enhanced adhesion of microorganisms to the pore area in the foam and environment for containing microorganisms, and a method for manufacturing the same.

According to the present invention, a method for manufacturing a foam includes the steps of: s1) kneading and extruding a resin composition including a base resin and a natural material to prepare an extrudate; and s2) pressurizing and foaming the extrudate to prepare a foam. The foam according to the present invention is prepared using the natural material and thus has excellent adhesion and affinity for microorganisms to the pore area in the foam and has the effect of greatly enhancing the environment for containing microorganisms. In particular, in the present invention, pressurized foaming (physical foaming) proceeds after the resin composition including the base resin and natural material is extruded, so that the above-described effects are achieved. Specifically, if extrusion and foaming proceed simultaneously or if pressurized foaming does not proceed after extrusion, it may be impossible to obtain a pore area of sufficient size by the natural material and to present adhesion and affinity of microorganisms to the foam. When the extrusion-foaming separated process, which includes step s1 of extruding the resin including the natural material, followed by step s2 of performing pressurized foaming, is performed, the extrudate with high structural stability and density is obtained in step s1 and, in step s2, the extrudate is thus effectively physically foamed so that internal pore activation and hydrophilization modification effect by the natural material are enhanced.

The natural material may be a compound, substance, or a mixture thereof, produced by an organism existing in nature. Specifically, the natural material may be a vegetable natural material, and more specifically, the natural material may be a cellulose-based natural material derived from plants. Examples of cellulose-based natural materials include, but are not limited to, plant stems, roots, leaves, fruits, skins, or mixtures thereof.

In an advantageous example, the vegetable natural material may be wheat bran, corn starch, or a mixture thereof.............. Specifically, wheat bran is the remainder of milled wheat from which flour and germ have been separated and has a form of powder. Wheat bran is mostly composed of the outer layers of the wheat kernels and contains a small amount of endosperm and germ. More specifically, the wheat bran consists of the seed coat of wheat, the core layer, the aleurone layer, and the like, and is composed of water, fat, protein, ash, and carbohydrates. Corn starch is a starch extracted from the endosperm of corn, and is the whitest and finest of all starches. Corn starch is a polymeric carbohydrate in which a large number of glucose monomers are linked by glycosidic bonds. The particles of corn starch are very hard crystalline, but when mixed with water and heated, they are loosened while absorbing moisture. Corn, which is a raw material for corn starch, is not particularly limited. As an example, corn, which is a raw material of corn starch, may be such a variety as, e.g., dent, hard (flint), sweet corn, popcorn, and soft corn and, according to the content of amylose, normal com (amylose of about 27%), waxy corn, high amylose corn (amylose of 60% or more), but is not limited thereto.

Wheat bran, corn starch or a mixture thereof is mixed with the hydrophobic base resin, and as pressurized-foamed after extrusion, the surface of the foam to which microorganisms may be attached is modified to be hydrophilic, so that the adhesion and affinity of microorganisms may be remarkably enhanced. Specifically, it is possible to significantly enhance the hydrophilicity of the foam, as compared with extrudates, through the two-step process which mixes the natural material and the base resin and extruding and then foaming the mixture. More specifically, in a case where wheat bran, corn starch, or a mixture thereof is used as the natural material, the water contact angle in the equilibrium state, which is measured by dropping a water droplet (deionized water of 2 ml) from a height of 1 cm on the macroscopically flat surface of the foam, is 10° or less, specifically 5° or less, more specifically 1° or less, and substantially 0°. As such, the foam may exhibit excellent hydrophilicity. Further, when wheat bran or corn starch is used as the natural material, the water contact angle is substantially 0°, and the time it takes for the water droplets to reach the equilibrium state is extremely short, e.g., less than 3 seconds. Thus, wheat bran or corn starch may have high hydrophilic surface properties than any other natural material. Further, when wheat bran, corn starch, or a mixture thereof is used as the natural material, it acts as an essential nutrient component necessary for anabolism of microorganisms attached to the foam, thereby increasing the concentration of microorganisms attached to the pores.

In step s1), the natural material may preferably have a moisture content of 10% by weight or less, specifically 0.1% by weight to 10% by weight, more specifically 0.1% by weight to 5% by weight, even more specifically, 0.1% by weight to 3% by weight. When the moisture content of the natural material is higher than the upper limit, extrusion and foaming may simultaneously occur as moisture is vaporized by a high temperature in the extrusion process of step s1). Accordingly, the probability of producing an irregular, random-shaped foam may increase, reproducibility is low, and the internal pore formation rate by physical pressure is significantly reduced, and durability may also be relatively reduced. In other words, in general, since natural materials have a moisture content of about 12% by weight or more, which exceeds 10% by weight, if a normal natural material is used as it is without controlling the moisture content, the above-described problems may occur. Specifically, if the moisture content of the natural material exceeds 10% by weight, unwanted foaming may occur in the extrusion step. Further, if foaming has already occurred partially in the extrusion step, not only is foaming rate reduced during physical foaming in step s2), but also durability may be reduced due to non-uniform foaming.

Means for controlling the moisture content of the natural material is not particularly limited, and an example thereof may be a drying means. In other words, the method for manufacturing the foam according to the present invention may further include the step of drying the natural material before step s1) to control the moisture content of the natural material. Any drying temperature and drying time may be adopted as long as the natural material may be thereby allowed to meet a required moisture content.

The method for manufacturing the foam according to an embodiment of the present invention may further include the step of treating the fiber surface of the natural material before step s1), more preferably, before the drying step. The fiber surface treatment may be a hydrophilization surface treatment of the fiber. As the hydrophilization surface-treated natural material is used, not only is it better dispersed in a dispersion medium, such as water in step s2), but also the outer surface of the foam and the surface of the internal pores of the foam are hydrophilized, allowing for more effective attachment of microorganisms. Specifically, when the hydrophilization surface treatment is performed in advance, the surface of the natural material is roughened, causing physical modification to increase specific surface area, simultaneously with chemical modification due to the introduction of acid groups on the surface. The physical and chemical modification may affect the melt-kneading process and the pressurized foaming process, thereby enhancing the adhesion of microorganisms to the pore area in the foam and leading a better containing environment.

An example of the hydrophilization surface treatment means is acid treatment. A specific example may be a means for drying the natural material after immersing the natural material in an aqueous solution containing an inorganic acid, such as sulfuric acid, hydrochloric acid, nitric acid or a mixed acid thereof for a predetermined time. The immersion temperature and immersion time are not particularly limited, but may preferably be in a low temperature range of 2°C to 14°C and in a range of 6 hours to 18 hours. The acid concentration of the aqueous solution is not particularly limited and may be, e.g., 20% by weight to 95% by weight. Further, ethanol may be further included in the aqueous solution, and in this case, it is possible to lead to more effective hydrophilization for the natural material.

In step s1), the weight ratio of the base resin and the natural material is not significantly limited. For example, the resin composition may preferably contain 0.1 parts by weight to 10 parts by weight of the natural material, specifically 0.5 parts by weight to 7 parts by weight, with respect to 100 parts by weight of the base resin. The foam prepared to meet the conditions may have a high specific surface area and secure sufficient structural stability.

In step s1), the base resin is not limited as long as it is a polymer capable of forming a foam and, as an example, the base resin may include any one or two or more selected from, e.g., polyolefin-based resins, polyvinyl chloride resins, polystyrene resins, ester resins, and copolymers thereof. As a specific example, the polyolefin-based resins may include polyethylene, polypropylene, and the like, and the copolymers may be any form of copolymers, such as a random form, an alternating form, and a block form. Although the base resin as described above has chemical hydrophobicity, the foam finally prepared by the above-described means has high hydrophilicity. As a preferable example, a poly(propylene-ethylene) random copolymer may be selected as the base resin.

As a more preferable example, in a case where a poly(propylene-ethylene) random copolymer is selected as the base resin and wheat bran, corn starch, or a mixture thereof is selected as the natural material, wheat bran and poly(propylene-ethylene) random copolymer are selected have much higher viscoelastic properties than wheat bran or corn starch while the poly(propylene-ethylene) random copolymer and wheat bran or corn starch, particularly wheat bran, have excellent adhesion properties to each other. Due to the above properties, the high viscoelasticity of the poly(propylene-ethylene) random copolymer may generate pores at a very high rate when the resin composition is pressurized and foamed after extrusion, and wheat bran or com starch, which has low viscoelastic properties, is mainly located on the inner wall of the pores due to high adhesion during the foaming process and is thus effective for adhesion and culture of microorganisms. The weight ratio of the weight of wheat bran located on the outer surface of the foam and the inner pore surface of the foam to the total weight of the wheat bran, corn starch, or a mixture thereof contained in the foam may be 60% by weight or more, preferably 70% by weight and, without limitations thereto, 95% by weight or less.

The melt flow rate (MFR) of the base resin is not particularly limited, and may be, e.g., 2 g/10 min to 20 g/10 min at 230°C, and the melting point (MP) may be 120 °C to 180 °C. Further, the weight average molecular weight of the base resin is not particularly limited, and may be, e.g., 5,000 g/mol to 1,000,000 g/mol. However, this is merely a specific example, and the present invention is not necessarily limited thereto.

In step s1), the extrusion temperature may be higher than the temperature at which the resin composition may be melted, and for example, the extrusion temperature may be in a melting range of the thermoplastic resin, e.g., from 130°C to 250°C.

In step s1), the shape and size of the obtained extrudate may be appropriately adjusted to suit the required shape and size of the final prepared foam. As a specific example, the shape may include various ones, such as a spherical shape, a rod shape, a barrel shape, a cylindrical shape, a cross shape, and an n-hedron (n is 4 or more). Examples of the size may be 0.1 cm to 5 cm, but it is not limited because it may be appropriately adjusted according to the scale of use.

In step s2), the pressurized foaming may be performed by introducing the extrudate, a foaming agent and a dispersion medium into a reactor and controlling the pressure in the reactor. As described above, in the present invention, since the above-described effects are obtained as the process of performing pressurized foaming after extrusion is carried out, pressurized foaming is one of the important components for implementing the effects. This physical foaming is performed by pressurization, and the extrudate containing the natural material is converted into a very low-density foam having internal pores (including open pores) due to the instantaneous change in pressure. In this case, the content of the extrudate, the foaming agent and the dispersion medium may be appropriately adjusted. For example, with respect to 100 parts by weight of the extrudate, 0.5 parts by weight to 30 parts by weight of the foaming agent and 50 parts by weight to 500 parts by weight of the dispersion medium may be used, but the present invention is not limited thereto.

In step s2), the foaming temperature and foaming pressure during pressurized foaming may be any temperature and pressure where the extrudate may be expanded and foamed, e.g., 100°C to 250°C. and 0.5 MPa to 10 MPa, respectively. However, this is merely a preferable example, and the present invention is not necessarily limited thereto.

In step s2), if the required foaming temperature and/or the required foaming pressure is reached after the inside of the reactor is sealed, the foaming temperature and foaming pressure are maintained for a predetermined time, so that the high temperature peak calorific value may be adjusted to be maintained. In this case, the maintaining time is not particularly limited, but may be, e.g., 3 minutes to 30 minutes. However, this is merely a preferable example, and the present invention is not necessarily limited thereto.

In step s2), the foaming agent may be any physical foaming agent that allows the extrudate to expand and foam due to a change in pressure. As a specific example, any one or two or more selected from inorganic physical foaming agents and organic physical foaming agents, such as aliphatic hydrocarbons or halogenated hydrocarbons, may be mixed and used. In this case, the aliphatic hydrocarbons may refer to C3 to C6 aliphatic hydrocarbons, such as n-butane, i-butane or n-pentane, and the halogenated hydrocarbons may refer to halogen-substituted aliphatic hydrocarbons, such as ethyl chloride, 2,3,3,3-tetrafluoro-1-propene or trans-1,3,3,3-tetrafluoro-1-propene. The inorganic physical foaming agents may include any one or two or more selected from, e.g., carbon dioxide, carbon monoxide, nitrogen, argon, helium, air, and the like. However, this is merely a preferable example, and the present invention is not necessarily limited thereto.

In an example of the present invention, in step s2), any one or two or more additives selected from a dispersing agent, a hydrophilic powder, and a foaming activator may be, together with the foaming agent and the dispersion medium, put in the reactor. As a specific example, the dispersion medium may be any medium capable of dispersing the extrudate, e.g., water or an organic solvent. Examples of the dispersing agent include a surfactant, EBS (ethylene bis stearamide)-based, montane-based, polyethylene-based waxes and metal stearate-based dispersing agent. A variety of known surfactants may be used, and as an example, anionic surfactants, such as sodium alkylbenzenesulfonate may be used. Further, the hydrophilic powder may include any one or two or more selected from, e.g., calcium carbonate, activated clay, aluminum hydroxide, slaked lime, zeolite, bentonite, diatomaceous earth, montmorillonite, illite, stilbite, kaolinite, pyrophyllite, andalusite, kyanite, miranite, grovenite, amesite, cordierite, feldspar, elephant metakaolin, hectorite, saponite, hectorite fluoride, beidellite, nontronite, stevensite, vermiculite, volkonskoite, soconite, magadite, kenyalite, smectite, attapulgite, sepiolite, halloysite and permutite. Further, the foaming activator may include any one or two or more selected from aluminum sulfate, potassium aluminum sulfate, aluminum ammonium sulfate, iron (III) sulfate, ferric polysulfate, sulfuric acid, sulfamic acid, sodium hydroxide and potassium hydroxide. The content of the additive used is not particularly limited, and for example, may be used in 0.001 parts by weight to 5 parts by weight or 0.1 parts by weight to 10 parts by weight, depending on the type of additive, with respect to 100 parts by weight of the extrudate.

The foam according to the invention may have an apparent density of 10 g/l to 180 g/l, specifically 10 g/l to 100 g/l.

The foam according to the present invention may include pores, and the volume ratio of the volume of the pores to the total volume of the foam may be 60% or more, specifically 70% or more, 80% or more, or 90% or more. and 99% or less.

In this case, the average diameter of the pores may be 20 µm to 300 µm, specifically 50 µm to 250 µm, and more specifically 80 µm to 250 µm.

As described above, the foam according to the present invention may be used for containing microorganisms, and more specifically, may be used for biochemical water treatment purposes. Specifically, as the foam may be used for biochemical water treatment purposes, it is possible to increase the treatment efficiency of the fluidized bed wastewater treatment device, reduce the treatment tank volume, and prevent the outflow of sludge due to fluctuations in the inflow wastewater flow rate. Further, the foam may be used for biochemical water treatment as a practically excellent carrier because of its high stability against fluctuations in wastewater quality and quantity.

In addition to the purpose for containing microorganisms, the foam according to the present invention may be applied, without limitation, in any field where a porous material may be used, that is, foamed plastic field. For example, the foam may be used for various purposes, such as insulating materials, fillers, buoyancy materials, and interior/exterior materials.

A wastewater treatment method using the foam according to the present invention may include the step of purifying wastewater by bringing the wastewater in contact with the foam.

Specifically, the wastewater treatment method using the foam according to an embodiment of the present invention may include a pretreatment step of precipitating and removing contaminants contained in wastewater using natural sedimentation and a purification step in which the treated water that has undergone the pretreatment step passes through a tube filled with the foam containing microorganisms.

When wastewater is treated through such a wastewater treatment method, a sufficient contact area between the microorganisms and the wastewater may be secured in the purification step, and the amount transmitted per unit time may increase. Thus, it is possible to treat a large amount of wastewater within a short time.

The pretreatment step may refer to a step of primarily removing solid contaminants contained in wastewater using, e.g., filtration. By going through the pretreatment step, it is possible to prevent a sharp reduction in purification efficiency when the solid contaminants are filtered through the foam upon later purification using microorganisms.

As a means for containing microorganisms in the foam, any typical method to contain microorganisms in a carrier may be used. For example, the methods known in Korean Patent No. 10-0945309, Korean Patent No. 10-1002696, or Korean Patent Application Publication No. 10-2008-0092091 may be used, and more specifically, a method for immersing a foam in a solution containing a microbial culture may be used.

Further, the wastewater treatment method using the foam according to another aspect of the present invention may include the purification step in which the wastewater containing microorganisms passes through the foam-filled tube. As described above, the foam may be used in a state in which the microorganisms are contained in the foam, but the foam may also be used in a state in which microorganisms are not contained. Specifically, the biochemical reaction of microorganisms by the foam, e.g., BOD reduction rate, may be significantly increased even when the wastewater containing the microorganisms subjected to biochemical treatment in an aerobic reactor passes through the foam-filled tube. In other words, the wastewater treatment method using the foam according to an aspect of the present invention may include the step of introducing microorganisms and wastewater into an aerobic reactor to performing biochemical treatment and a purification step in which the wastewater containing the biochemically treated microorganisms passes through the foam-filled tube.

Further, the wastewater treatment method according to an embodiment of the present invention may further include a washing step of washing the foam after the purification step. Through such washing, it is possible to remove substances, such as suspended matter existing in the foam, and it is possible to secure high wastewater treatment efficiency in the long term. The foam that has undergone the washing step may be subjected to the purification step again. The washing step and the purification step may be alternately performed.

When the foam according to the present invention fills the filling tube, the porosity, which is the ratio of the empty space to the total volume of the foam-filled tube, may be 30% to 60%.

As the microorganisms, any microorganisms capable of biochemical treatment in the field of wastewater treatment may be used, which may include ay one or two or more selected from, e.g., Bacillus sp., Leuconostoc sp., Lactobacillus sp., Lactococcus sp., Streptococcus sp., Aspergillus sp., Saccharomyces sp., Pseudomonas sp., and Candida sp. However, this is merely a specific example, and the present invention is not necessarily limited thereto.

Hereinafter, the present invention is described in detail in connection with embodiments thereof, but the scope of the present invention is not limited by the following embodiments.

### Embodiments of the present invention

### (Embodiment 1)

### <Kneading and extrusion process of wheat bran, a natural material>

First, wheat bran was dried at 50°C to have a moisture content of 3% by weight or less.

990 g of a propylene-ethylene random copolymer having a melt flow rate (MFR, at 230°C) of 7 g/10 min and a melting point (MP) of 140°C and 10 g of wheat bran having a moisture content of 3 wt% or less and an average particle size of 20 *µ*m were mixed and melt-kneaded at 170°C. The molten composition was supplied to a twin-screw extruder having an inner diameter of 20 mm, and was extruded into strand shapes while being introduced into a water tank and cooled. The cooled extrudate was cut to about 2 mg and dried to obtain resin particles. In this case, as extrusion conditions, a raw material supply part of the extruder was filled with the molten composition, and extrusion was carried out while supply of the resin was adjusted by a capacitive feeder so that the discharge amount of the raw material resin by the same number of screw rotations is not more than that for the filled operation condition, for the typical discharge amount of raw material resin when the filled operation condition. Here, the discharge amount during the gear operation with respect to the filled operation was adjusted to 70%. The discharged resin particles were produced into cross-shaped tiny pellets (mini-pellets) by the shape of a discharge part.

### <Pressurized foaming process>

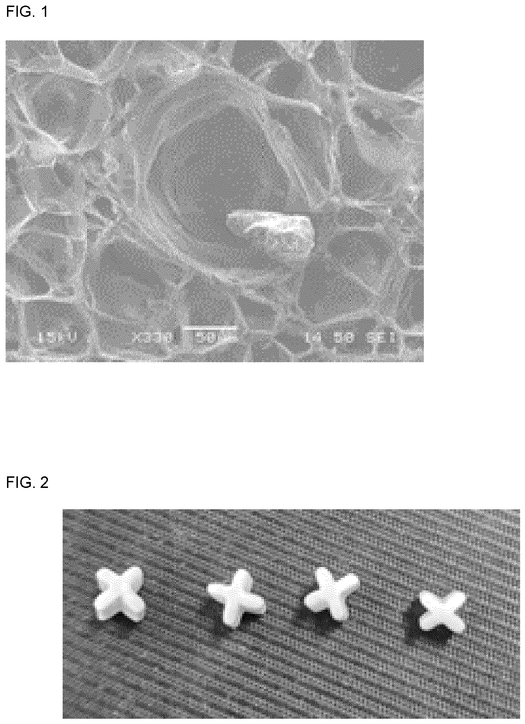

3 kg of the resin particles were dispersed in 3 kg of water in a sealed container equipped with a stirrer, and 10 g of kaolin, 2 g of sodium alkylbenzenesulfonate, and 1 g of aluminum sulfate were added during dispersion. Further, 80 g of carbon dioxide was supplied into the sealed container, and the temperature was raised to 130°C to adjust the pressure in the sealed container to 6.0 Mpa, and then left for 15 minutes. The sealed container was then opened and the content was discharged under atmospheric pressure, producing a foam having an apparent density of 45 g/l. FIG. 1 is a photograph of a foam (cross-shaped tiny pellets) prepared according to embodiment 1, taken on a scanning electron microscope. FIG. 2 is an optical photograph of a foam prepared according to embodiment 1.

### (Embodiment 2)

A foam was prepared using the same method as that in embodiment 1 except that 970 g of a propylene-ethylene random copolymer and 30 g of wheat bran were used in the kneading and extrusion processes.

### (Embodiment 3)

A foam was prepared using the same method as that in embodiment 1 except that 950 g of a propylene-ethylene random copolymer and 50 g of wheat bran were used in the kneading and extrusion processes.

### (Embodiment 4)

A foam was prepared using the same method as that in embodiment 1 except that wheat bran acid-treated by an acid treatment process described below was used in the kneading and extrusion processes.

### <Acid treatment process>

The natural material was immersed in a mixture containing 20 ml of ethanol and 15 ml of 0.5 M aqueous sulfuric acid solution, irradiated with ultrasonic waves at 25°C for 1 hour, and then dried at 50°C, obtaining an acid-treated natural material having a moisture content of 3% or less.

### (Embodiment 5)

A foam was prepared using the same method as that in embodiment 1 except that wheat bran having a moisture content of 12.15%, instead of wheat bran having a moisture content of 3% or less, were used in the kneading and extrusion processes.

### (Embodiment 6)

### <Kneading and extrusion process of corn starch, a natural material>

First, corn starch having a moisture content of 3% or less was prepared by drying at 50°C.

The corn starch and a propylene-ethylene random copolymer having a melt flow rate (MFR, at 230°C) of 7 g/10 min and a melting point (MP) of 140°C were sufficiently kneaded, and a masterbatch in which 900 g of the copolymer and 100 g of cornstarch were mixed was prepared.

Then, 50 g of the masterbatch was melt-kneaded at 170°C. The molten composition was supplied to a twin-screw extruder having an inner diameter of 20 mm, and was extruded into strand shapes while being introduced into a water tank and cooled. The cooled extrudate was cut to about 2 mg and dried to obtain resin particles. In this case, as extrusion conditions, a raw material supply part of the extruder was filled with the molten composition, and extrusion was carried out while supply of the resin was adjusted by a capacitive feeder so that the discharge amount of the raw material resin by the same number of screw rotations is not more than that for the filled operation condition, for the typical discharge amount of raw material resin when the filled operation condition. Here, the discharge amount during the gear operation with respect to the filled operation was adjusted to 70%. The discharged resin particles were produced into cross-shaped tiny pellets (mini-pellets) by the shape of a discharge part.

### <Pressurized foaming process>

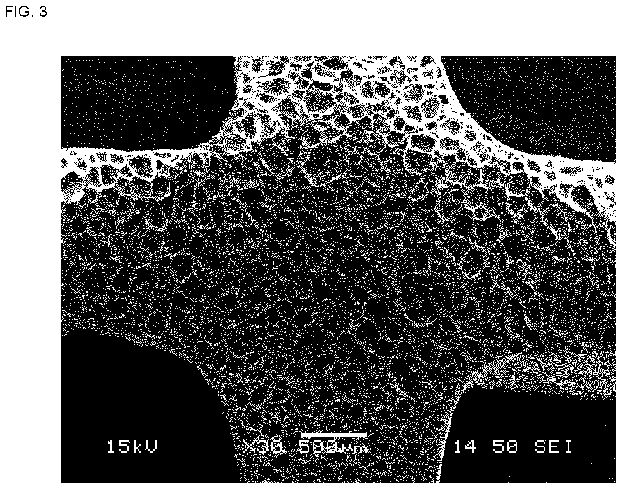

3 kg of the resin particles were dispersed in 3 kg of water in a sealed container equipped with a stirrer, and 10 g of kaolin, 2 g of sodium alkylbenzenesulfonate, and 1 g of aluminum sulfate were added during dispersion. Further, 80 g of carbon dioxide was supplied into the sealed container, and the temperature was raised to 130°C to adjust the pressure in the sealed container to 6.0 Mpa, and then left for 15 minutes. The sealed container was then opened and the content was discharged under atmospheric pressure, producing a foam having an apparent density of 45 g/l. FIG. 3 is a photograph of a foam (cross-shaped tiny pellets) prepared according to embodiment 6, taken on a scanning electron microscope. From comparison between FIGS. 1 and 3, it may be identified that corn starch leads to formation of more uniform pores throughout the pellet.

### [Comparative Example 1]

A foam was prepared using the same method as that in embodiment 1 except that wheat bran having a moisture content of 12.15%, instead of wheat bran having a moisture content of 3% or less, were used in the kneading and extrusion processes and the pressurized foaming process was not performed. In this case, as wheat bran having a moisture content not less than 10% by weight, it is possible to foam the resin particles by the moisture of the wheat bran even by extrusion in the atmospheric atmosphere.

### [Comparative Example 2]

A foam was prepared using the same method as that in embodiment 1 except that wheat bran was not used.

### [Experimental Example 1]

### <Evaluation of BOD reduction rate of foam>

Aerobic reactors having a capacity of 100L were filled with the foams of embodiments 1 to 5 and comparative example 1 in 25 volume%, and initial operation was started at 20°C on wastewater having a biochemical oxygen demand (BOD) of 300 ppm. The BOD reduction rate when the hydraulic retention time (HRT) was three hours by controlling the inflow and outflow flow rates was measured. The results are shown in Table 1 below. BOD measurement was performed based on what is stipulated in the Enforcement Regulations of the Environmental Conservation Act.

**[Table 1]**

| | Embodiment 1 | Embodiment 2 | Embodiment 3 | Embodiment 4 | Embodiment 5 | Comparison Example 1 |
|---|---|---|---|---|---|---|
| BOD reduction rate (%) | 91.3 | 92.9 | 94.1 | 98.2 | 84.1 | 60.8 |

In Table 1, it may be identified from embodiments 1 to 3 that the BOD reduction rate increases as the content of wheat bran increases. It may also be identified from embodiment 4 that when acid-treated wheat bran is used instead of normal wheat bran, the BOD reduction rate is significantly increased. The results are believed to come from the fact that the surface of the foam, such as the outer surface of the foam and the inner surfaces of the pores of the foam, where microorganisms may be attached, is hydrophilized by mixing wheat bran with the hydrophobic base resin. Furthermore, when sulfuric acid-treated wheat bran is used, it may be identified that better results are obtained, which are believed to come from the fact that the physical modification in which sulfuric acid treatment roughens the surface of wheat bran to increase the specific surface area and chemical modification according to the introduction of sulfonic acid groups on the surface affect the melt-kneading and pressurized foaming processes to enhance the adhesion of microorganisms to the pore area in the foam and the containing environment.

In embodiment 5, as compared to embodiments 1 to 4, a low BOD reduction rate was shown. This is believed to be due to the fact that, as wheat bran with a high moisture content is mixed with the base resin in the kneading and extrusion process, foaming proceeds in the extrusion process, lowering the structural stability and durability of the resin particles. Specifically, it was identified that in embodiments 1 to 4, foaming did not occur at all during the extrusion process but, in embodiment 5, foaming occurred even during extrusion. In the extrusion process, the foaming by the high moisture content of the wheat bran significantly lowers the effect of increasing internal pores and specific surface area as compared to the foaming by pressurized foaming. Further, the so foamed resin particles are significantly inferior in structural stability and durability as compared to non-foamed resin particles in which wheat bran having a low moisture content is used. Therefore, when the resin particles foamed by the high moisture content of wheat bran during the extrusion process are then pressurized and foamed, the probability of producing an irregular random foam may increase, the reproducibility is low, the rate of formation of internal pores by physical pressurization is significantly reduced, and the durability is relatively reduced. From these results, a lower moisture content in the wheat bran, as a specific example, 10% by weight or less, 5% by weight or less, preferably 3% by weight or less, may be preferable.

On the other hand, comparative example 1 showed a significantly reduced BOD reduction rate as compared to the embodiments. This is believed to be due to the fact that as pressurized foaming is not performed, the area of containing microorganisms in the foam is not significantly increased. From these results, it may be shown that physical foaming needs to be performed on wheat bran in the base resin to greatly affect the enhancement of the adhesion of microorganisms in the pore area in the foam and the containing environment. Embodiment 6 is a result of preparing an eco-friendly foam using corn starch, and shows a BOD reduction rate similar to those obtained in embodiments 1 to 3 in which wheat bran was used.

### [Experimental Example 2]

### <Evaluation of mechanical properties of foam>

The pore size, porosity and structural stability of the foams of embodiments 1 to 4 and comparative example 1 were evaluated. As a result, in embodiments 1 to 4 in which wheat blood was used, there was substantially no significant decrease in structural stability, compared to comparative example 2 in which wheat blood was not used, and it was identified that the pore size and porosity of the foam were superior.

### [Experimental Example 3]

### <Evaluation of water contact angle of foam>

After dropping 2 ml of deionized water droplets at a height of 1 cm on each of the flat surfaces of the foams of embodiments 1 to 6 and comparative examples 1 and 2, the time taken to reach the equilibrium and the water contact angle in the equilibrium state were measured. In measuring the water contact angle, the time taken to reach the equilibrium state (hereinafter, referred to as "stabilization time") and water contact angle by randomly dropping droplets of deionized water on ten random areas of the flat surface of the foam were measured, and their respective average values were taken.

The foams prepared in embodiments 1 to 5 and 6 all had a water contact angle of 0°, indicating that the foams were completely wetted in water. For the foams prepared in embodiments 1 to 3, the stabilization time was about 2.4 seconds to about 2.8 seconds, and the stabilization time became shorter as the content of wheat bran increased. For the foams prepared in embodiment 4, it was identified that the stabilization time was about 1.2 seconds indicating that it had the highest hydrophilic surface properties. For the foam prepared in embodiment 5, it was identified that the water contact angle was 0° but a significant difference in stabilization time (2 seconds to 5 seconds) occurred depending on the position of the droplet compared to the foams prepared in the other embodiments. Thus, it may be identified that the foam of embodiment 5 has a hydrophilic surface but the surface properties are non-uniform and the degree of hydrophilicity is relatively low. For the foam prepared in embodiment 6, the stabilization time was about 2.7 seconds and, given the relative content of corn starch relative to the resin, it may be identified that wheat bran exhibits better hydrophilic surface properties. The samples prepared in comparative examples 1 and 2 exhibited a water contact angle of 10° to 15°.

## Claims

1. A method for manufacturing a foam, the method comprising the steps of:
s1) preparing an extrudate by kneading and extruding a resin composition including a base resin and a natural material; and
s2) preparing the foam by pressurized foaming the extrudate.

2. The method of claim 1, wherein
in step s1), the natural material is at least one selected from the group consisting of a plant stem, a plant root, a plant leave, a fruit, and a fruit skin.

3. The method of claim 1, wherein
in step s1), the natural material includes wheat bran, corn starch, or a mixture thereof.

4. The method of claim 1, wherein
in step s1), the natural material has a moisture content of 10% by weight or less.

5. The method of claim 4, further comprising
the step of drying the natural material before step s1).

6. The method of claim 1, wherein
in step s1), the resin composition includes 0.1 parts by weight to 10 parts by weight of the natural material relative to 100 parts by weight of the base resin.

7. The method of claim 1, wherein
in step s1), the base resin includes any one or two or more selected from the group consisting of a polyolefin-based resin, a polyvinyl chloride-based resin, a polystyrene-based resin, a polyester resin, and a copolymer thereof.

8. The method of claim 4, further comprising
the step of performing fiber surface treatment on the natural material before step s1.

9. The method of claim 1, wherein
in step s2), the pressurized foaming is performed by introducing the extrudate, a foaming agent, and a dispersion medium into a reactor and controlling a pressure in the reactor.

10. The method of claim 9, wherein
in step s2), during the pressurized foaming, a foaming temperature is 100°C to 250°C, and a foaming pressure is 0.5 Mpa to 10 Mpa.

11. The method of claim 9, wherein
in step s2), the foaming agent includes a physical foaming agent of any one or two or more selected from the group consisting of carbon dioxide, carbon monoxide, nitrogen, argon, helium, butane, and pentane.

12. The method of claim 1, wherein
the foam is a foam for containing microorganisms.

13. A foam manufactured by the method for any one of claims 1 to 12.

14. The foam of claim 13, wherein
the foam includes pores, and wherein microorganisms are contained in the pores.

15. A method for purifying wastewater using a foam, the method purifying the wastewater by bringing the wastewater in contact with a foam manufactured by the method of any one of claims 1 to 12.
